# EUROPÄISCHE PATENTANMELDUNG

(11) **EP 0 660 111 A2**
(43) Veröffentlichungstag der Anmeldung: **28.06.1995**
(21) Anmeldenummer: 94119362.5
(22) Anmeldetag: 08.12.1994
(51) Int. Cl.: G01N 33/24, G01N 3/00, G01N 15/08

(54) **Einrichtung zur Messung von Formstoffeigenschaften**

(30) Priorität: 23.12.1993 CH 3842/93
(71) Anmelder: GEORG FISCHER GIESSEREIANLAGEN AG, CH-8201 Schaffhausen (CH)
(72) Erfinder: Kruse, Ernst Otto, D-78244 Gottmadingen (DE); Renner, Christian, CH-8222 Beringen (CH)
(74) Vertreter: Szilagyi, Marianne

(57) **Zusammenfassung**

Die Einrichtung zur Messung von Eigenschaften bei Giesserei-Formstoffen weist eine Press-Vorrichtung (2) mit einem Press-Stempel (6) auf, an welchem für weitere Prüfungen an Prüfkörpern (11, 18) ein Scher-Stempel (17) ein Biege-Druck-Stempel (20) und ein Ein- oder Mehrfach-Press-Stempel (21) anbringbar sind. Auf die Auflage (8) der Pressvorrichtung (2) sind unterschiedliche Auflageteile (9, 15, 16, 19) aufsetzbar. Ausserdem weist die Einrichtung eine Vorrichtung (4) zur Messung der Gasdurchlässigkeit, eine Gewichtsmess-Vorrichtung (3) zur Bestimmung des Schütt- und Prüfkörpergewichtes auf. Die Einrichtung ist ferner zur Messung der Zugfestigkeiten an Prüfkörpern ausgebildet und erfasst alle Messwerte On-Line.

## Beschreibung

Die Erfindung betrifft eine Einrichtung zur Messung von Formstoffeigenschaften, wie sie im Oberbegriff von Anspruch 1 gekennzeichnet ist.

Durch die CH-A-646 790 ist eine Einrichtung der eingangs genannten Art bekanntgeworden, welche lediglich zur Prüfung der Druckfestigkeit, Biegefestigkeit und der Verdichtbarkeit von Giesserei-Formstoffen geeignet ist.

Für die Beurteilung der Formstoffeigenschaften sind jedoch weitere Prüfungen bzw. Messungen erforderlich. Für derartige Messungen wie z.B. Schüttgewicht, Prüfkörpergewicht, Gasdurchlässigkeit, Doppelscherfestigkeit, Grünzugfeistigkeit, Nasszugfestigkeit, Spaltfestigkeit und Biegefestigkeit sind für jede Prüfung einzeln Geräte bekanntgeworden, wie sie z.B. in einem Prospekt "Prüfgeräte für Form- und Kernsande" der Firma Georg Fischer Giessereianlagen AG, Prospekt Nr. GA 293/1a (8.91) beschrieben sind.

Alle diese Geräte haben den Nachteil, dass sie immer nur für eine Art von Prüfung verwendbar sind, und auch nur jeweils einen Messwert liefern.

Lediglich bei dem durch die CH-A-646 790 gekanntgewordenen Einrichtung können mehrere Messungen vorgenommen werden, jedoch sind die zusätzlich angeführten Messungen bzw. Prüfungen mit den weiteren Geräten erforderlich um die Eigenschaften eines Giessereiformstoffes einwandfrei beurteilen zu können.

Die Erfindung hat die Aufgabe diesen Nachteil zu beheben und nur eine Einrichtung der eingangs genannten Art vorzuschlagen, mit welcher die wichtigen Formstoffeigenschaften von bentonit- und kunstharzgebundenen Formstoffen gemessen bzw. geprüft werden können, wobei auch eine Kostensenkung und eine Platzersparnis im Prüfungslabor erreicht werden soll.

Die Erfindung hat weiterhin die Aufgabe, dass alle Daten QS-gerecht, d.h. On-Line erfasst und protokolliert werden.

Erfindungsgemäss wird diese Aufgabe durch eine Einrichtung der eingangs genannten Art gelöst, welche ferner die im Kennzeichen des Anpruches 1 aufgeführten Merkmal aufweist.

Besonders vorteilhafte Ausbildungen der Erfindung sind in den davon abhängigen Ansprüchen gekennzeichnet.

Die Erfindung ist in der beiliegenden Zeichnung vereinfacht beispielsweise dargestellt und nachfolgend beschrieben. Es zeigen:
- Fig. 1: eine Seitenansicht mit Teilschnitten der Einrichtung zur Messung von Formstoffeigenschaften
- Fig. 1a: Vorrichtungsteile zur Spalt- und Druckfestigkeitsmessung eines Prüfkörpers
- Fig. 1b: Vorrichtungsteile zur Doppelscherfestigkeitsmessung
- Fig. 1c: Vorrichtungsteile zur Biegefestigkeitsmessung
- Fig. 1d: Vorrichtungsteile zur Herstellung von Biege-Prüfkörpern und
- Fig. 2: eine Frontansicht der Einrichtung von Fig. 1.

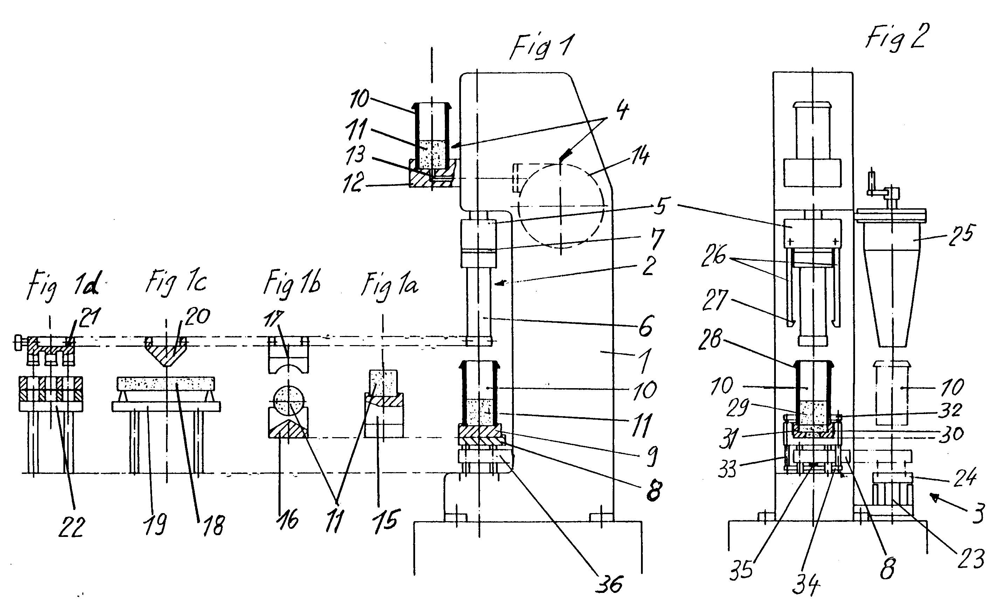

Die in den Fig. 1 und 2 gezeigten Einrichtung weist einen Ständer 1 auf, an welchem eine Pressvorrichtung 2, eine Gewichtsmessvorrichtung 3 und eine Vorrichtung 4 zur Messung der Gasdurchlässigkeit angeordnet ist. Die Pressvorrichtung 2 weist einen vorzugsweise elektrisch oder aber auch hydraulisch bzw. pneumatisch betätigbaren Stellantrieb 5 mit einem Press-Stempel 6 auf, wobei zwischen dem Press-Stempel 6 und dem Stellantrieb 5 eine Druckmesseinrichtung 7, wie z.B. eine elektrische Signale erzeugende Druckmessdose, angeordnet ist.

Ausserdem ist der Stellantrieb 5 mit einer weiter nicht dargestellten Wegmesseinrichtung wirkverbunden. Die Pressvorrichtung 2 weist einen am Ständer 1 angeordneten Schwenkarm 36 mit einer Auflage 8 auf, welche zur Aufnahme unterschiedlicher Auflageteile ausgebildet ist. Fig. 1 zeigt ein Auflageteil 9 zur Aufnahme einer Hülse 10, welche mit dem zu prüfenden Giesserei-Formstoff gefüllt wird. Durch den Press-Stempel 6 wird der Formstoff verdichtet, wobei die Messung der Verdichtbarkeit durch eine Kraft- und eine Wegmessung erfolgt.

Der gemessene Verdichtungsweg bestimmt gleichzeitig die Höhe eines Prüfkörpers 11. Falls dieser nicht innerhalb eines Toleranzbandes die Normhöhe aufweist, wird ein neuer mit einer entsprechend geänderten Formstoff-Einfüllmenge hergestellt.

Die Vorrichtung 4 zur Messung der Gasdurchlässigkeit weist eine am Ständer 1 angeordnete Auflageplatte 12 auf, welche eine Düse 13 aufweist, die mit einem im oder am Ständer 1 angeordneten Gasdruckerzeuger 14 - vorzugsweise ein Drucklufterzeuger - wirkverbunden ist. Zur Messung der Gasdurchlässigkeit wird der Prüfkörper 11 mit der Hülse 10 auf die Auflageplatte 12 gestellt, wobei durch Messung des Druckes bzw. des Druckabfalles die Gasdurchlässigkeit des verdichteten Formstoffes bestimmt wird.

Zur Prüfung der Spalt- und Druckfestigkeit wird ein aus Fig. 1a ersichtlicher Auflageteil 15 mit dem Prüfkörper 11 auf die Auflage 8 des Ständers gestellt, wobei die Messung bei der Zerstörung des Prüfkörpers 11 durch den Press-Stempel 6 mit der Druckmesseinrichtung 7 erfolgt.

Die Fig. 1b zeigt ein weiteres Auflageteil 16 für den Prüfkörper 11 zum Aufstellen auf der Auflage 8 und einen Scherstempel 17 zur Befestigung am Stempel 6, wobei diese Teile zur Messung der Doppelscherfestigkeit ausgebildet sind.

Zur Messung der Biegefestigkeit an einem vorzugsweise aus Kernsand bzw. Kunstharzsand bestehenden stabförmigen Probekörper 18 wird ein in Fig. 1c dargestelltes Auflageteil 19 am Ständer 1 und ein Biege-Druck-Stempel 20 am Stempel 6 befestigt.

Die Herstellung von Probestäben 18 erfolgt mit dem in Fig. 1d dargestellten Mehrfach-Press-Stempel 21 und der entsprechenden Press-Form 22, welche ebenfalls am Stempel 6 bzw. am Ständer 1 anbringbar sind. Der Press-Stempel 21 und die Pressform 22 können auch als Einfach-Stempel bzw. Einfach-Form ausgebildet sein.

Die Gewichtsmess-Vorrichtung 3 weist seitlich am Ständer 1 eine Waage 23 auf, über deren Auflagetisch 24 am Ständer 1 eine Fülltrichter 25 angeordnet ist. Die Hülse 10 wird mit ihrem Auflageteil 9 mit dem Schwenkarm 36 auf die Waage 23 unter den Fülltrichter 25 geschwenkt.

Die vollständig mit Formstoff gefüllte Hülse 10 wird gewogen, wodurch das Schüttgewicht bestimmbar ist.

Nach dem Verdichtungsvorgang wird die Istlänge gemessen und damit die Verdichtbarkeit ermittelt. Aus der Differenz Istlänge zu Normlänge des Prüfkörpers 11 und dem Schüttgewicht wird das Sollgewicht für den runden Normprüfkörper aus bentonitgebundenem Formstoff berechnet. Dies entspricht dem sogenannten Prüfkörpergewicht.

Wie aus Fig. 2 ebenfalls ersichtlich, sind an der Press-Vorrichtung 2 bzw. an dessen Stellantrieb 5 seitlich des Press-Stempels 6 schwenkbare Greifer 26 angeordnet, welche in Vorsprünge 28 der Hülse 10 einrastbare Zughaken 27 aufweisen.

Für die Messung der Zugfestigkeit ist ein Prüfkörper 29 mit einer im Durchmesser grösser werdenden Endpartie erforderlich. Hierfür wird die Hülse 10 auf ein, einen hinterschnittenen Hohlraum 31 aufweisenden Bodenteil 30 aufgesteckt und auf die Auflage 8 gestellt.

In die Hülse 10 und den Bodenteil 30 wird Formstoff eingefüllt, wobei durch Verdichten mittels des Press-Stempels 6 der Prüfkörper 29 hergestellt wird. Eine oberhalb des Bodenteils 30 angeordnete Anschlagplatte 32 ist mittels Stangen 33 mit einem unterhalb der Auflage 8 angeordneten Joch 34 verbunden, wobei zwischen dem Joch 34 und der Auflage 8 eine Druckmesseinrichtung 35 angeordnet ist.

Zur Messung der Grün- und der Nasszugfestigkeit werden die Greifer 26 abgesenkt bis sie mit den Zughaken in die Vorsprünge 28 der Hülse 10 einrasten.

Dann wird die Hülse 10 mit dem Bodenteil 30 und dem Prüfkörper 29 mittels des Stellantriebes 5 nach oben gezogen. Nachdem das Bodenteil 30 an der Anschlagplatte 32 zur Anlage gelangt, wird der Prüfkörper 29 auf Zug beansprucht. Das Joch 35 wird hierbei über die verschiebbaren Stangen 33 gegen die fest angeordnete Druckmesseinrichtung 35 gedrückt, wodurch die Zugkraft bis zum Erreichen der Zugfestigkeit beim Zerreissen des Prüfungskörpers 29 gemessen wird.

Zur Messung der Nass-Zugfestigkeit wird ein beheizbares Bodenteil 30 verwendet, wodurch der Aufheizvorgang der Form beim Giessen simuliert wird. In der dabei entstehenden Kondensationszone verringert sich die Festigkeit des Formstoffes.

Die ermittelten Messwerte werden einem Auswertgerät als elektrische Signale zugeleitet und dort angezeigt und/oder ausgedruckt.

Durch die beschriebene einfache und von Hand bedienbare Einrichtung können alle erforderlichen Messungen bzw. Prüfungen an Formstoff -Prüfkörpern vorgenommen werden, wobei eine einfache und rasche Umrüstung der Einrichtung für die verschiedenen Messungen gewährleistet ist. Alle, die Messung beeinflussenden Bewegungen (Verdichtvorgang, Prüfvorgang) sind motorisch angetrieben, die Geschwindigkeit ist vom Bediener nicht beeinflussbar. Alle Messwerte werden On-Line erfasst.

Da für alle Messungen nur eine Einrichtung mit einem Auswert- und Anzeigegerät erforderlich ist, ergeben sich geringe Investitionskosten und ein geringer Platzbedarf.

## Patentansprüche

1. Einrichtung zur Messung von Formstoffeigenschaften an aus Giesserei-Formstoff bestehenden Prüfkörpern mit einer, einen Press-Stempel und einer Auflage aufweisenden Pressvorrichtung zur Messung der Verdichtbarkeit der Spalt- und der Druckfestigkeit, dadurch gekennzeichnet, dass die Einrichtung zusätzlich fest angeordnete und anbringbare bzw. auswechselbare Vorrichtungen aufweist, mittels welchen zusätzlich eine Messung des Schüttgewichtes, der Gasdurchlässigkeit, der Doppel-Scherfestigkeit, der Zugfestigkeit und der Biegefestigkeit durchführbar ist.

2. Einrichtung nach Anspruch 1, dadurch gekennzeichnet, dass an der Pressvorrichtung (2) je nach Messart jeweils ein Scher-Stempel (17) mit einem Auflageteil (16), ein Biege-DruckStempel (20) mit einem Auflageteil (19) für einen stabförmigen Probekörper (18) oder ein Ein- oder Mehrfach-Press-Stempel (21) mit einer entsprechenden Press-Form (22) zur Herstellung von Probekörpern (18) anbringbar sind.

3. Einrichtung nach Anspruch 1 oder 2, dadurch gekennzeichnet, dass seitlich der Pressvorrichtung (2) eine Gewichtsmess-Vorrichtung (3) mit einem Fülltrichter (25) und einer Waage (24) zur Gewichtsmessung des in einer Hülse (10) eingefüllten Formstoffes angeordnet ist.

4. Einrichtung nach einem der Ansprüche 1 bis 3, dadurch gekennzeichnet, dass eine mit einer Düse (13) versehene Auflageplatte (12) für den Prüfkörper (11) angeordnet ist, welche mit einem Gasdruckerzeuger (14) zur Messung der Gasdurchlässigkeit wirkverbunden ist.

5. Einrichtung nach einem der Ansprüche 1 bis 5, dadurch gekennzeichnet, dass an der Pressvorrichtung (2) Greifer (26) angeordnet sind, welche in Vorsprünge (28) der Hülse (10) einrastbare Zughaken (27) aufweisen.

6. Einrichtung nach einem der Ansprüche 1 bis 5, dadurch gekennzeichnet, dass in die Auflage (8) ein mit einem hinterschnittenen Hohlraum (31) versehenes Bodenteil (30) einsetzbar ist, welches mit der aufsetzbaren Hülse (10) die Form zur Herstellung eines Prüfkörpers (29) zur Messung der Grünzugfestigkeit bildet.

7. Einrichtung nach Anspruch 6, dadurch gekennzeichnet, dass zur Messung der Nasszugfestigkeit das Bodenteil (30) beheizbar ist.

8. Einrichtung nach einem der Ansprüche 5 bis 7, dadurch gekennzeichnet, dass das Bodenteil (30) mittels den in die Hülse (10) eingreifenden Greifern (26) gegen eine Anschlagplatte (32) vertikal verschiebbar ist, wobei ein mit der Anschlagplatte (32) verbundenes Joch (34) gegen eine fest angeordnete Druckmesseinrichtung (35) zur Messung der Zugfestigkeit drückbar ist.
